# EUROPÄISCHE PATENTSCHRIFT

(11) **EP 2 676 017 B1**
(45) Veröffentlichungstag und Bekanntmachung des Hinweises auf die Patenterteilung: **22.05.2019**
(21) Anmeldenummer: 12702992.4
(22) Anmeldetag: 07.02.2012
(51) Int. Cl.: F01N 11/00, F01N 3/20, F01N 9/00

(54) **VERFAHREN ZUR MODELLBASIERTEN BESTIMMUNG DER TEMPERATURVERTEILUNG EINER ABGASNACHBEHANDLUNGSEINHEIT**
METHOD FOR MODEL-BASED DETERMINATION OF THE TEMPERATURE DISTRIBUTION OF AN EXHAUST GAS POST-TREATMENT UNIT
PROCÉDÉ DE DÉTERMINATION, BASÉ SUR UN MODÈLE, DE LA RÉPARTITION DE LA TEMPÉRATURE D'UNE UNITÉ DE TRAITEMENT DE GAZ D'ÉCHAPPEMENT

(30) Priorität: 16.02.2011 DE 102011011426; 27.05.2011 DE 102011103346
(43) Veröffentlichungstag der Anmeldung: 25.12.2013
(73) Patentinhaber: MTU Friedrichshafen GmbH, 88045 Friedrichshafen (DE)
(72) Erfinder: HEHLE, Marc, 78464 Konstanz (DE); MÜLLER, Ralf, 88693 Deggenhausertal (DE); NIEMEYER, Jens, 88045 Friedrichshafen (DE); REMELE, Jörg, 88709 Hagnau (DE); SCHÄFFNER, Guido, 88263 Horgenzell (DE); SINZENICH, Holger, 88677 Markdorf (DE); SPÄDER, Tim, 88045 Langenargen (DE)
(74) Vertreter: Kordel, Mattias
(86) Internationale Anmeldenummer: PCT/EP2012/000558
(87) Internationale Veröffentlichungsnummer: WO 2012/110210

(56) Entgegenhaltungen:
- EP-A1- 2 025 388
- DE-A1- 10 036 942
- DE-A1- 10 305 451
- DE-A1-102006 021 303

## Beschreibung

Die Erfindung betrifft ein Verfahren zur modellbasierten Bestimmung der Temperaturverteilung einer Nachbehandlungseinheit für Abgase, insbesondere eines Katalysators, bevorzugt eines SCR-Katalysators oder eines Partikelfilters, gemäß dem Oberbegriff des Anspruches 1.

Ein solches Verfahren ist beispielsweise aus der DE 103 05 451 A1 und aus der DE 100 36 942 B4 bekannt und geht davon aus, dass die über die Durchströmungslänge einer Abgasnachbehandlungseinheit, zum Beispiel eines Katalysators, ermittelte mittlere Temperatur jedenfalls im dynamischen Betrieb, und der damit verbundenen inhomogenen Temperaturverteilung, keine hinreichende Basis dafür abgibt, das System Motor-Abgasnachbehandlungseinheit so abzustimmen, dass die strengen Anforderungen an die Abgasqualität mit der erforderlichen Genauigkeit eingehalten werden. Dem soll dadurch genügt werden, dass die Abgasnachbehandlungseinheit modellbasiert in axialer Richtung in eine Anzahl von Scheiben aufgeteilt wird und dass die Temperatur jeder Scheibe als Funktion der Temperatur des die Scheibe anströmenden Gases bestimmt wird. Dies unter der Annahme, dass die radiale Temperaturverteilung konstant ist und eine adiabatische Wärmeübertragung zwischen dem Abgas und den Scheiben der Abgasnachbehandlungseinheit gegeben ist.

Diese Annahmen führen wiederum zu gewissen Ungenauigkeiten, insbesondere durch den entgegen der Annahme sich ausbildenden radialen Temperaturgradienten, weswegen vorgesehen wird, diese Wärmeverluste überschlagsmäßig zu erfassen und auch zu berücksichtigen.

Bei einem weiteren aus der DE 10 2009 046 771 A1 bekannten Verfahren zur modellbasierten Bestimmung der Temperaturverteilung von Abgasnachbehandlungseinheiten wird zunächst der axiale Temperaturverlauf zumindest bereichsweise bestimmt und es wird aufbauend hierauf, wiederum zumindest bereichsweise, ein mehrdimensionales Temperaturkennfeld auf Basis einer analytischen Beziehung berechnet. Dieser können verschiedene Randbedingungen, wie beispielsweise eine bereits bekannte Temperatur und/oder Eigenschaften der Abgasnachbehandlungseinheit zugrunde gelegt werden, welche für den Wärmetransport von Bedeutung sind. Aus dem berechneten Temperaturkennfeld kann die positionsabhängige Temperatur entnommen werden, womit durch den axialen Temperaturverlauf und das Temperaturkennfeld jeweils zumindest eine an einer beliebigen Position innerhalb der Abgasnachbehandlungseinheit gegebene Temperatur vorliegt.

Bei der DE 10 2006 021 303 B4 ist es bezogen auf die Erfassung der Temperaturverteilung in einer Abgasnachbehandlungseinheit bekannt, die axiale Temperaturverteilung mittels eines dynamischen Wärmemodells zu bestimmen und die während der Abgasnachbehandlung generierte Reaktionswärme, die aus der Konvertierung von Abgasbestandteilen resultiert, mittels eines kinematischen Modells zu erfassen. Dies, um die Qualität der ermittelten Temperaturverteilung bei möglichst geringem Rechenaufwand zu verbessern. Durch die Erfassung der radialen, senkrecht zur Hauptströmung gegebenen Temperaturverteilung lässt sich dabei fallweise eine weitere Qualitätsverbesserung erreichen, was aber wiederum einen höheren Rechenaufwand bedingt.

Die DE 103 47 132 A1 offenbart ein Verfahren zur Mengenabschätzung von in einem harnstoffbasierten SCR-Katalysator gespeicherten Ammoniak auf Basis eines dynamischen Katalysator-Modells und generiert die Schätzung auf der Grundlage von gemessenen und geschätzten Werten, wobei hierfür über stromauf und stromab des Katalysators angeordnete NO_{X}- und Temperatur-Sensoren Messwerte zur Verfügung gestellt werden, die bezogen auf den stromab des Katalysators liegenden NO_{X}-Sensor aufgrund der Querempfindlichkeit gegenüber Ammoniak auch eine Aussage über den Ammoniakanteil im Abgas stromab des Katalysators ermöglichen.

In entsprechender Weise sind bei der EP 2 025 388 A1 Sensoren für die Erfassung von Messwerten an einer Abgasreinigungseinheit eingesetzt, die mit einem SCR-Katalysator arbeitet, der bezüglich der Zudosierung und Speicherung von Ammoniak in Berücksichtigung der Messwerte modellbasiert geregelt ist.

Aus der DE 10 2007 045 263 A1 ist ein Verfahren zur Steuerung der Reduktionsmittelzufuhr in ein Abgasnachbehandlungssystem eines Verbrennungsmotors mit einem SCR-Katalysator, einem SCR-Speichermodell, einer Steuereinheit und einer Dosiervorrichtung bekannt, das eine Optimierung der dem Katalysator zugeführten Reduktionsmittelmenge unabhängig von ausschließlich nach der Reduktionsreaktion bestimmbaren Messgrößen ermöglichen soll und durch das, bezogen auf den SCR-Katalysator, ein Reduktionsmitteldurchbruch unbedingt vermieden werden soll. Für die Steuereinheit sind als Eingangsgrößen Stickoxid-Rohemission, Speichertemperatur und Speicherfüllstand erfasst. Basierend hierauf erfolgt die Berechnung der maximal möglichen Konvertierungsrate sowie die Berechnung des als Reduktionsmittel verbrauchten Ammoniaks. Hiervon ausgehend ergibt sich die über den Katalysator austretende Menge an Stickoxiden und Ammoniak sowie ein Regelsignal, das, rückgekoppelt auf das SCR-Speichermodell, über das Speichermodell die Größe der über die Dosiereinrichtung einzudüsenden Menge an Reduktionsmittel beeinflusst und das zusammen mit der im Speichermodell berücksichtigten Eindüsungsmenge über das Speichermodell für den Speicherfüllstand berücksichtigt wird, der eine Eingangsgröße zur Steuereinheit bildet.

Die DE 10 2010 025 382 A1 zeigt einen SCR-Katalysator, auf den stromauf das Reduktionsmittel eingedüst wird und dessen Katalysatorkörper analog zur DE 100 36 942 B4 quer zur Durchströmungsrichtung in Scheiben unterteilt ist. Dies, um für die Funktion des Katalysators wesentliche Parameter sowohl scheibenweise erfassen zu können wie auch für das Gesamtergebnis wesentliche Änderungen in den aufeinander folgenden Scheiben, insbesondere in Bezug auf deren Fähigkeit zur Einspeicherung von Reduktionsmittel, für das Gesamtergebnis nutzen zu können.

Durch die Erfindung soll in Berücksichtigung der Temperaturverteilung über die Länge der Abgasnachbehandlungseinheit modellbasiert das System Motor-Abgasnachbehandlungseinheit so abgestimmt werden, dass bei vertretbarem Aufwand die Einhaltung vorgegebener Abgasgrenzwerte, insbesondere der gesetzlich vorgegebenen Abgasgrenzwerte, im Regelfall gewährleistet ist.

Erreicht wird dies mit den Merkmalen des Anspruches 1. Die Unteransprüche geben zweckmäßige Weiterbildungen an. Weitere erfindungsgemäße Einzelheiten ergeben sich aus der Beschreibung und den Zeichnungen.

Die Erfindung geht bezüglich der Abgasnachbehandlungseinheit, bevorzugt eines Katalysators, insbesondere eines SCR-Katalysators, für die modellbasierte Bestimmung der Temperaturverteilung und die damit bei einem SCR-Katalysator in Zusammenhang stehende NH₃-Speicherfähigkeit von der Aufteilung der Abgasnachbehandlungseinheit in mehrere Scheiben quer, insbesondere senkrecht zur Durchströmungsrichtung aus. Für jede dieser Scheiben wird die Temperatur und die Gaskonzentration im Modell gerechnet, wobei, bezogen auf die Durchströmungsrichtung, die Ausgangswerte der jeweils vorausgehenden Scheibe als Eingangswerte für die nachfolgende Scheibe dienen.

Für jede der Scheiben wird in bekannter Weise eine Mengenbilanz durchgeführt. Die Resultate dieser Mengenbilanzen werden dazu verwendet, bezogen auf das Beispiel des SCR-Katalysators als Abgasnachbehandlungseinheit, den NH₃-Speicherfüllstand zu ermitteln und zu beeinflussen. Grundsätzlich kann durch ein solches thermisches Modell das Volumen des SCR-Katalysators und die darin gespeicherte NH₃-Menge besser genutzt und bei der Dosierung des Reduktionsmittels, also bezogen auf den SCR-Katalysator der NH₃-Menge, berücksichtigt werden, so dass sich NH₃-Durchbrüche bei optimierter Umsatzrate vermeiden lassen.

Die Aufteilung in Scheiben ermöglicht somit, bezogen auf einen SCR-Katalysator, scheibenbezogen die virtuelle Erfassung der NH₃- und/oder NO_{X}-Konzentration entsprechend einem virtuellen Sensor. Durch die räumliche Auflösung der NH₃-Speichermenge, des NO_{X}-Umsatzes und der NH₃-Oxidation, entsprechend der im Modell erfolgten Auflösung des Katalysatorkörpers in Scheiben, wird bei transienten Bedingungen das NH₃-Speichervolumen besser genutzt und der NH₃-Schlupf vermieden.

Entsprechend zur scheibenweisen axialen Temperaturberechnung und der Kontrolle mittels Messung einer Ein- und Ausgangstemperatur zur Abgasnachbehandlungseinheit, also insbesondere einem SCR-Katalysator, wird die radiale Temperaturverteilung der Scheibe modelliert. Hierzu wird jede Scheibe virtuell, entsprechend der axialen Aufteilung des Katalysatorkörpers in Scheiben, in radialer Richtung zerlegt, sei es in zueinander konzentrische Ringe, Quader oder anderweitig geformte, insbesondere regelmäßig geformte Segmente, die sich zu dem jeweiligen scheibenförmigen Körper ergänzen. Die Rechenmethode zur Temperaturermittlung entspricht jener beim axialen Scheibenmodell, wobei, bezogen auf die ohnehin gemessene Umgebungstemperatur, die sich zum Umfang ergebenden Temperaturverluste berücksichtigt werden.

Für die Kontrolle und Kalibrierung der berechneten radialen Temperaturverteilung ist zweckmäßigerweise neben der eintritts- und austrittsseitig vorgesehenen Temperaturmessung, insbesondere über Temperatursensoren, eine zusätzliche Temperaturmessung austrittsseitig vorgesehen, über die in austrittsseitig radial zum Umfang beabstandeten Bereichen die Temperatur erfasst wird, insbesondere dadurch, dass mindestens zwei in verschiedenen radialen Positionen vorgesehene Temperatursensoren angeordnet sind. Anstelle einer solchen zusätzlichen, austrittsseitigen Temperaturmessung kann auch mit nur einem Sensor gearbeitet werden, wenn dieser durch seine Positionierung zum Katalysator in einer Lage angeordnet ist, in der eine entsprechende mittlere Temperatur vorliegt, beispielsweise in einem größeren axialen Abstand zum Katalysator.

Erreicht wird durch die kombiniert radiale und axiale Temperaturmodellierung eine optimierte Nutzung der jeweiligen Abgasnachbehandlungseinheit, so beispielsweise bei Katalysatoren des Potenzials zu einer Reduzierung des Bauraums, und bei Partikelfiltern mit aktiver Regeneration durch die damit verbundene optimierte Regenerationsweise auch die Einsparung von Kraftstoff.

Die axiale und radiale Temperaturberechnung bezogen auf die jeweiligen Scheiben des im Modell unterteilten Körpers der Abgasnachbehandlungseinheit erfolgt bei der erfindungsgemäßen Lösung unter Berücksichtigung des radialen Wärmeübergangs auf den Umfang der Nachbehandlungseinheit mittels eines Wärmeübergangswiderstandes R_{C} und bei der Berechnung des Wärmeübergangs vom Abgas auf das Material der Abgasnachbehandlungseinheit, also die Segmente der Nachbehandlungseinheit, unter Berücksichtigung einer Wärmeübergangszahl k.

Diese Art der modellbasierten Bestimmung der Temperaturverteilung der Abgasnachbehandlungseinheit bietet erfindungsgemäß den besonderen Vorteil, durch das Vorgehen bei der modellbasierten Bestimmung der Temperaturverteilung auch Ausgangswerte zur Verfügung zu stellen, die im Modell in Abhängigkeit davon, ob sich die vor der Nachbehandlungseinheit in vorgegebenen Zeitabständen gemessenen Abgastemperaturen gegenüber einem zeitlichen Mittelwert derselben ändern, zwischen einem stationären und einem instationären Arbeitsbetrieb unterscheiden.

In der Erfindung wird dabei davon ausgegangen, dass ein stationärer Arbeitsbetrieb vorliegt, wenn die vor der Abgasnachbehandlungseinheit, insbesondere in regelmäßigen Zeitabständen, gemessene Abgastemperatur keine größeren, also oberhalb eines vorgegebenen Schwellwertes liegenden Abweichungen zu einem zeitlichen Mittelwert zeigt. Der Schwellwert kann variabel, also in Abhängigkeit von weiteren Randbedingungen festgelegt werden und liegt bevorzugt beispielsweise bei mindestens 1°K, kann aber auch innerhalb eines ein Mehrfaches desselben ausmachenden Bereiches liegen. Ergeben sich bezogen auf diesen stationären Arbeitsbetrieb für die nach der Abgasnachbehandlungseinheit gemessene mittlere Temperatur von der gemittelten Modelltemperatur nach der Abgasnachbehandlungseinheit unterhalb des Schwellwertes liegende Unterschiede, so erfolgt eine Anpassung des Modells, und zwar dadurch, dass der Wärmeübergangswiderstand R_{C} geändert wird, bis Übereinstimmung besteht. Im beschriebenen stationären Arbeitsbetrieb erfolgt somit die Anpassung des Modells an den tatsächlichen Zustand und dies über eine Veränderung des Wärmeübergangswiderstandes R_{C}.

Das Temperaturmodell für eine Abgasnachbehandlungseinheit stellt sich insbesondere als SCR-Temperaturmodell, somit für den vorstehend angesprochenen stationären Arbeitsbetrieb als ein Regelkreis dar, der durch Veränderung des Wärmeübergangswiderstandes R_{C} abgestimmt werden kann, um den eingeschwungenen Zustand zu erreichen. In der Praxis häufig auftretende kurzzeitige Änderungen der Überströmungsverhältnisse zur Abgasnachbehandlungseinheit, insbesondere zum Katalysator, bleiben so im Wesentlichen ohne Einfluss auf das Modell.

Anders liegt der Fall, wenn, insbesondere bei Laständerungen entsprechender Größe, sich die Betriebsgegebenheiten für die Abgasnachbehandlungseinheit - gegebenenfalls zusätzlich - dahingehend ändern, dass sich Abweichungen der vor der Abgasnachbehandlungseinheit, also beispielsweise vor dem Katalysator, gemessenen Abgastemperatur gegenüber einem zeitlichen Mittelwert ergeben. Für diesen Fall wird davon ausgegangen, dass der in der Gewichtung geringere Wärmeübergangswiderstand R_{C} während des vergleichsweise kurzen Einschwingvorganges gleich bleibt, den geänderten Gegebenheiten aber durch eine Änderung der Wärmeübergangszahl k Rechnung getragen werden kann, die von der Strömungsgeschwindigkeit des Abgases, und damit der Last abhängig ist.

In welche Richtung der k-Wert, also die Wärmeübergangszahl zu ändern ist, ergibt sich aus den gemessenen und errechneten Temperaturverläufen über der Zeit, ermittelt aus den mittleren Temperaturen nach Abgasnachbehandlungseinheit, und den ersten beiden Ableitungen hierzu. Abhängig von der relativen zeitlichen Lage der durch Maxima bzw. Minima bestimmten Wendepunkte in der Ableitungskurve wird der k-Wert vergrößert oder verkleinert.

Ungeachtet dessen, dass der die Bedingungen zur Umgebung der Nachbehandlungseinheit berücksichtigende Wärmeübergangswiderstandswert R_{C} im instationären Fall, also für den als instationär erfassten Zeitraum, als konstant betrachtet wird, bleibt die geänderte Wärmeabgabe an die Umgebung auch im instationären Fall berücksichtigt, da sich aufgrund der Änderung der inneren Temperaturen der Scheiben ein geänderter Wärmefluss in radialer Richtung ergibt.

Die nach Abgasnachbehandlungseinheit, also vor allem nach dem Katalysator, insbesondere dem SCR-Katalysator, gegebene gemittelte Modelltemperatur wird dadurch ermittelt, dass die für Bezugsflächen, also beispielsweise Ringe, der letzten Scheibe gerechneten Temperaturen entsprechend deren Flächenanteilen gemittelt werden. Weiter wird davon ausgegangen, dass die Wärmeübergangszahl k jedenfalls für bestimmte Lastzustände und entsprechende Abgasströmungszustände als bekannt - und auch korrekt - vorausgesetzt werden kann, so dass für den stationären und für den instationären Fall diesbezüglich von der gleichen Sachlage ausgegangen werden kann und für die Anpassung des Modells - im instationären Fall durch Veränderung des k-Wertes - eine jeweils gleiche Bezugsbasis gegeben ist. Im instationären Fall kann über die Anpassung des SCR-Modells hinaus die zuzuführende NH₃-Menge geändert und/oder gegebenenfalls auch in die Regelung der Brennkraftmaschine eingegriffen werden.

Der SCR-Katalysator ist im Rahmen der Erfindung bevorzugt in eine Regelstruktur eingebunden, die neben dem SCR-Modell mit einer Vorsteuerung und einem Regler arbeitet und die gegebenenfalls auch zur Durchführung eines Modellabgleiches einzusetzen ist. Insbesondere dient die Regelstruktur zur Adaption der über das SCR-Modell erfassten Speicherkapazität bei Lastwechseln unter der Prämisse, eine ausreichende Umsatzrate durch Anpassung der Speicherfähigkeit im SCR-Modell sicherzustellen und die erforderliche Umsatzrate der Realität schnell anzupassen.

Eine Änderung der Speicherfähigkeit des SCR-Modells ist in der Regel nur bei Lastwechseln mit großer Temperaturänderung erforderlich. Für die Erfassung der Speicherfähigkeit wird das jeweilige maximale Speichervermögen und die augenblickliche Beladung der Katalysatorscheiben in Zeitschritten fortlaufend im SCR-Modell berechnet. Damit liefert das SCR-Modell die Grunddaten für die Entscheidung, ob eine Adaption im Sinne einer Einspeicherung durch Erhöhung der NH₃-Beladung oder einer Ausspeicherung durch Reduzierung der NH₃-Beladung vorzunehmen ist. Die Entscheidung auf Basis dieser Grunddaten, und damit in Abhängigkeit von der Speicherfähigkeit, ergibt sich aus einem Vergleich des jeweils vorgegebenen Emissionswertes, insbesondere des als Emissionswert vorgegebenen gesetzlichen Emissionsgrenzwertes, für NOₓ und dem im Modell berechneten NOₓ-Wert, der in der Vorsteuerung durchgeführt wird.

So ist beispielsweise bei einem NOₓ-Wert aus der Modellrechnung, der über dem vorgegebenen Emissionswert als Sollwert liegt, eine Adaption durchzuführen, da im Katalysator zu wenig umgesetzt wird und durch die Erhöhung der dosierten NH₃-Menge, also durch Einspeichern von NH₃, ein auf den vorgegebenen Emissionswert abgestimmter Sollumsatz zu erreichen ist. Für die Einspeicherung wird über die Vorsteuerung die einzuspritzende NH₃-Menge entsprechend angehoben.

Generell wird über die Vorsteuerung die einzuspritzende NH₃-Menge so festgelegt, dass der jeweils vorgegebene Emissionswert, insbesondere ein oft länderspezifischer, gesetzlich vorgegebener Emissionsgrenzwert für NOₓ in Höhe von zum Beispiel 0,67g/kWh, eingehalten wird, wobei entsprechend der erkannten Abweichung des im Modell errechneten NOₓ-Wertes vom vorgegebenen Emissionswert die im SCR-Modell eingespeicherte NH₃-Menge vergrößert oder verkleinert wird. Somit wird beispielsweise bei einem zu großen Umsatz von NOₓ und einem daraus in der Vorsteuerung errechneten NOₓ-Wert, der unterhalb des vorgegebenen Emissionswertes liegt, die Einspritzung von NH₃ über die Vorsteuerung zurückgenommen, also mangels Zufuhr von NH₃ der Verbrauch an NH₃ durch Ausspeicherung von NH₃ abgedeckt.

Bei kleineren Abweichungen, in der Regel also im stationären Betrieb, wird davon ausgegangen, dass das SCR-Modell hinsichtlich des Speichervermögens der Scheiben richtig eingestellt ist. In diesem Fall gleicht der Regler durch schrittweise Anpassung der jeweiligen Dosiermenge den nach SCR gemessenen Istwert an den berechneten NOₓ-Sollwert nach Katalysator an. Die Abweichungen entstehen zum Beispiel durch Dosierungsungenauigkeiten.

Über den Regler wird somit bei kleineren Abweichungen der im SCR-Modell errechneten NOₓ-Werte von den gemessenen NOₓ-Werten nach SCR-Katalysator eine Anpassung der eingedüsten NH₃-Masse überlagert und unabhängig von den Festlegungen der Vorsteuerung gegebenenfalls vorgenommen, um eine schnelle Anpassung im Hinblick auf die Einhaltung der NOₓ-Emissionswerte zu gewährleisten.

Die Eingangsgrößen, die geliefert vom SCR-Modell in der Vorsteuerung verarbeitet werden, sind zumindest in der Hauptsache: NO nach SCR ppm, NO₂ nach SCR ppm, maximal umsetzbares NO mol/s, maximal umsetzbares NO₂ mol/s, NO umgesetzt mol/s, NO₂ umgesetzt mol/s, NH₃ max.mol speicherbar, NH₃ mol gespeichert.

In dem in die Regelstruktur integrierten Modellabgleich wird zu definierten Zeitpunkten der zeitliche Verlauf der Differenz zwischen dem vorgegebenen NOₓ-Emissionswert und dem entsprechenden im Modell errechneten NOₓ-Wert erfasst. Stimmen diese Werte überein, decken sich also die über der Zeit aufgetragenen NOₓ-Kurven dieser Werte, ist das Modell in Bezug auf die NH₃-Speicherfähigkeit und die NOₓ-Umsatzraten richtig eingestellt. Decken sich die Kurven nicht, so ist die Fläche zwischen den Kurven ein Maß für den Modellfehler. Übersteigt dieser Modellfehler einen Schwellwert, wird das Modell bezüglich der Speicherfähigkeit geändert.

Da sich die Speicherfähigkeit mit der Katalysatoralterung nur langsam ändert, erfolgt der Modellabgleich bevorzugt nur beim Ausspeichern, zumal der Modellfehler beim Ausspeichern im Vergleich zum Einspeichern deutlicher sichtbar wird. Findet ein Modellabgleich statt, so wird im SCR-Modell die durch die Alterung bedingte Änderung der Speicherfähigkeit miterfasst, so dass erfindungsgemäß auch sicherzustellen ist, dass trotz der durch die Alterungsdrift sich vermindernden maximalen Speicherfähigkeit für NH₃ über der Betriebsdauer eine ausreichende maximale Umsatzrate gewährleistet ist.

Im Rahmen der Erfindung werden im SCR-Modell die NOₓ-Reaktionen als chemische Reaktionen für die SCR-Steuerung/-regelung bezogen auf Harnstoff als Reduktionsmittel über die bekannten, nachstehend wiedergegebenen Hauptreaktionen erfasst.
1.) NO + NO₂ + 2 NH₃ → 2 N₂ + 3 H₂O,
   als schnelle Reaktion
2.) 4 NO + 4 NH₃ + O₂ → 4 N₂ + 6 H₂O
   als Standardreaktion, und
3.) 6 NO₂ + 8 NH₃ → 7 N₂ + 12 H₂O,
   als langsame Reaktion.

Für das Katalysatormaterial in Prüfstandserprobung ermittelte Kennfeldwerte für Umsätze und Speicherfähigkeit von NH₃ bilden eine wesentliche Grundlage für das SCR-Modell und die in diesem erfassten Abläufe.

Darauf basierend erfolgt die Ermittlung des Gesamtumsatzes in Stickoxiden, so des nach Katalysator gegebenen NOₓ-Wertes, wobei bezogen auf die einzelnen, in der Modellierung gegebenen Katalysatorscheiben die Strömungsgeschwindigkeit, die Temperatur, die NOₓ-Konzentration und die in der Scheibe verfügbare NH₃-Menge insbesondere vorrangig Berücksichtigung finden.

Weitere Einzelheiten und Merkmale der Erfindung ergeben sich aus den Ansprüchen, den nachfolgenden Erläuterungen und den Zeichnungen. Die Zeichnungen veranschaulichen:
- Fig. 1: eine schematische Darstellung eines virtuell axial in Scheiben gegliederten SCR-Katalysators, als Beispiel auch für durch anderweitige Katalysatoren und/oder Partikelfilter gebildete Abgasnachbehandlungseinheiten,
- Fig. 2 und 3: quer zur Durchströmungsrichung im Querschnitt segmentierte Katalysatorscheiben,
- Fig. 4: eine schematisierte Darstellung der thermischen Abläufe im SCR-Modell,
- Fig. 5: eine schematische und zusammenfassende Darstellung einer erfindungsgemäßen Regelstruktur in einem Ablaufdiagramm,
- Fig. 6: eine Prinzipdarstellung zur dynamischen Korrektur bezogen auf die thermischen Abläufe im SCR-Modell,
- Fig. 7: eine Übersicht der Regelstruktur zu einem erfindungsgemäßen modellbasiert geregelten SCR-Katalysator, und
- Fig. 8 und 9,: Darstellungen zur graphischen Ermittlung der Ein- und Ausspeichermenge von NH₃ bei über den Modellabgleich korrigierend ein- oder auszuspeichernder NH₃-Menge.

Aus der Praxis bekannt und vielfach auch in der Literatur, insbesondere der Patentliteratur beschrieben, sind vor allem mit Dieselmotoren arbeitende Antriebssysteme, bei denen nachgeordnet zum Motor Abgasnachbehandlungsanlagen eingesetzt sind, über die gewisse Abgasbestandteile, insbesondere im Abgas enthaltene Schadstoffe weitmöglichst aus dem Abgas entfernt oder zumindest unschädlich gemacht werden sollen. Zur Verminderung der im sauerstoffreichen Abgas von DieselBrennkraftmaschinen enthaltenen Stickoxide kommt insbesondere die sogenannte SCR-Technologie in Einsatz, bei der die Stickoxide mithilfe von Ammoniak oder einer entsprechenden, zu Ammoniak umsetzbaren Vorläufersubstanz selektiv zu Stickstoff und Wasser reduziert werden.

Damit dies mit einer hohen Konvertierungsrate für die Stickoxide erreicht wird, ist insbesondere die temperaturabhängige NH₃-Speicherfähigkeit des Katalysators zu berücksichtigen, die sich vor allem in Abhängigkeit von den Betriebsbedingungen der Brennkraftmaschine, aber auch den Umgebungsbedingungen und über der Durchströmungslänge des Katalysators ändert. Diese Änderungen sind real, insbesondere mit vertretbarem Aufwand, nicht zu erfassen. Deswegen wird parallel zur messtechnischen Erfassung der Temperaturen eingangsseitig und ausgangsseitig zum Katalysator die Temperaturverteilung in diesem virtuell modellbasiert erfasst, um in Berücksichtigung der messtechnisch erfassten Temperaturwerte und der virtuell ermittelten Temperaturwerte ein möglichst genaues Bild über die Temperaturverteilung zu erhalten und insbesondere in Berücksichtigung derselben die Zudüsung des Reduktionsmittels, also bevorzugt einer Ammoniak bildenden Substanz, auch in Korrelation zum Speicherverhalten des Katalysators steuern und/oder regeln zu können.

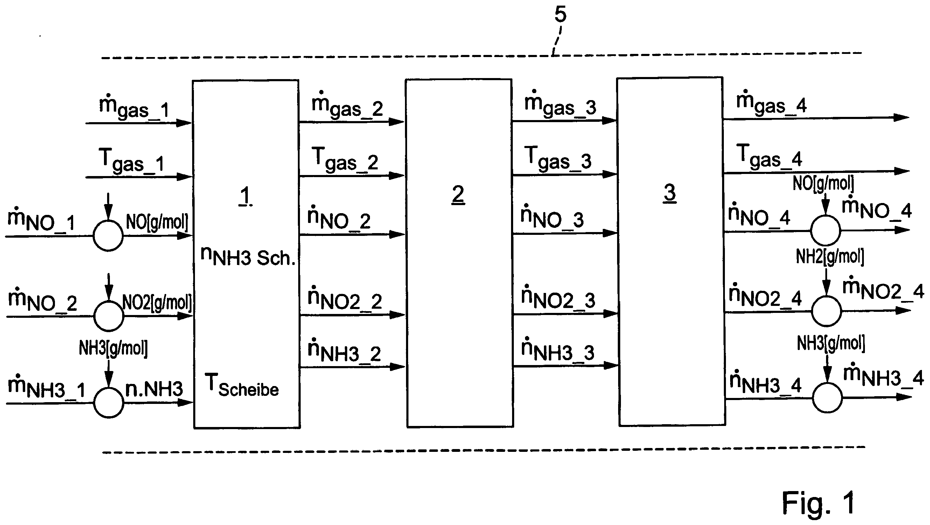

In Fig. 1 ist die im Modell gegebene Segmentierung des Katalysatorkörpers durch eine axiale Untergliederung in eine größere Anzahl von Scheiben durch die Darstellung von drei in Durchströmungsrichtung des Katalysators hintereinander liegenden Scheiben 1 bis 3 veranschaulicht und mit 5 eine zum Katalysatorkörper umfangsseitig umschließende Ummantelung strichliert angedeutet. Entsprechend dem über den Katalysator geleiteten Abgasstrom sind die Scheiben 1 bis 3 jeweils mit einem diesem Abgasstrom entsprechenden Massenstrom mgas beaufschlagt, bei sich ändernder Temperatur Tgas für die jeweilige Scheibe 1 bis 3.

Entsprechend der Motoremission und der Eindüsung von Reduktionsmittel in den Abgasstrom enthält dieser Massenanteile ***m*** von NO, NO₂ und NH₃, zu denen entsprechende Stoffmengenanteile n korrelieren, die sich entsprechend den jeweiligen Umsatzraten über dem Durchlauf durch die Scheiben 1 bis 3 ändern. Entsprechend der Darstellung stellen die Ausgangswerte der einen Scheibe, zum Beispiel Scheibe 1, die Eingangswerte der nächstfolgenden Scheibe, zum Beispiel der Scheibe 2 dar. In Berücksichtigung der in den Scheiben jeweils stattfindenden Reaktionen ergibt sich für jede der Scheiben eine entsprechende Temperatur TScheibe sowie auch die in der Scheibe eingespeicherte Stoffmenge nNH₃ in mol, wobei der Temperatureffekt aus den Reaktionen in den Scheiben im Vergleich zum Wärmeeintrag aus dem Abgasstrom wesentlich kleiner ist.

In Verfeinerung der Temperaturerfassung für die Scheiben kann, was nicht dargestellt ist, auch die Wärmeleitung zwischen aufeinander folgenden Scheiben erfasst werden. Die gesamte, im Katalysator eingespeicherte Stoffmenge an NH₃ entspricht der Summe der in den Scheiben eingespeicherten Stoffmengen nNH₃.

In Fig. 1 ist der axiale Temperaturverlauf über dem Katalysator über die Scheiben modelliert und zunächst für jede der Scheiben eine jeweilige Temperatur TScheibe ermittelt, die radiale Temperaturverteilung über jede der Scheiben aber als konstant angenommen. Auch in radialer Richtung ergibt sich aber real, nunmehr jeweils innerhalb der jeweiligen Scheibe, eine Temperaturschichtung mit Abfall der Temperatur gegen die Ummantelung 5, für die die Umgebungstemperatur im Regelfall deutlich unterhalb der Temperatur des Katalysators liegt.

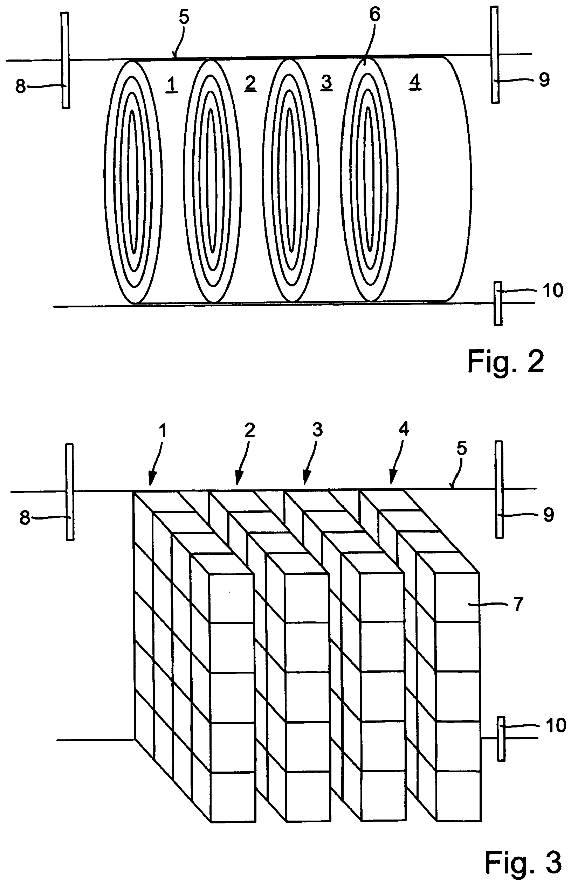

Wird, wie bei der Erfindung, für jede der virtuellen Scheiben die radiale Temperaturverteilung nicht konstant angenommen, so bedingt dies für jede der Scheiben 1 bis 4 in sich eine Unterteilung virtuell in Segmente, wie dies in den Fig. 2 und 3 im Schema veranschaulicht ist. Fig. 2 zeigt diesbezüglich virtuelle Segmente 6, die radial einander umschließen, wobei in der Darstellung ein zentrales Segment von einer größeren Anzahl von Ringsegmenten umschlossen ist.

Eine andere Art der Segmentierung veranschaulicht Fig. 3, wobei im Gegensatz zur Darstellung in Fig. 2 keine Ummantelung 5 in Ringform, sondern eine viereckige Ummantelung 5 vorgesehen ist, entsprechend deren quer zur Durchströmungsrichtung viereckigem Querschnitt die Scheiben 1 bis 4 virtuell in Quader 7 segmentiert sind.

In den Fig. 2 und 3 ist bezogen auf den jeweils im Modell segmentierten Katalysatorkörper zur Erfassung der anströmseitig und abströmseitig zum Katalysatorkörper gegebenen Temperatur des Abgasstromes die Anordnung von Temperaturerfassungsgliedern, insbesondere Sensoren 8, 9, veranschaulicht.

Bezogen auf die im Modell vorgesehene radiale Segmentierung und die mit dieser verbundene Möglichkeit der Erfassung der radialen Temperaturverteilung über der jeweiligen virtuellen Scheibe kann real ausgangsseitig zum Katalysator eine Temperaturerfassung in unterschiedlichen radialen Bereichen erfolgen, etwa, wie in Fig. 3 gezeigt, über zwei ausgangsseitig in verschiedenen radialen Positionen angeordnete Sensoren 9 und 10. Eine gemittelte ausgangsseitige Temperatur lässt sich dadurch bestimmen, dass die für die Segmente errechneten Temperaturen entsprechend ihren Flächenanteilen erfasst und gemittelt werden. Eine Alternative hierzu ist die sensorische Erfassung der Abgastemperatur in einem Abstand zum Katalysator, in dem sich die austretenden Abgase bereits gemischt haben und eine mittlere Temperatur vorliegt, die über einen Sensor zu erfassen ist.

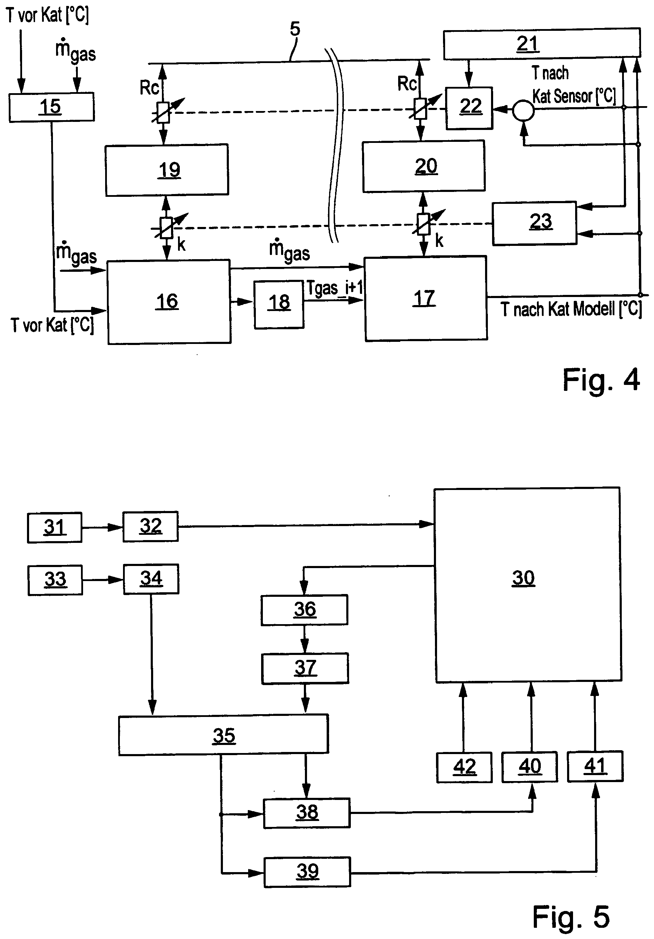

Eine erfindungsgemäße Möglichkeit zur Berücksichtigung der axialen und radialen Temperaturverteilung ist in Fig. 4 veranschaulicht und bietet insbesondere Vorteile auch hinsichtlich der Verwirklichung einer modellbasierten Regelstruktur, die nachstehend noch angesprochen wird.

Fig. 4 stellt ein thermisches Modell eines SCR-Katalysators in seinen Grundzügen dar, bei dem entsprechend Fig. 1 die Temperatur T vor KAT und der Abgas-Massenstrom ***ṁ*** gas die Eingangsgrößen bilden, die über eine Polstellenkompensation 15 zum rechnerischem Ausgleich der Sensorträgheit - und bevorzugt auch verifiziert und auf ihre Plausibilität überprüft - einem fiktiven Wärmespeicher Gasmasse 16 zugeführt werden, in dem der Wärmeinhalt der Gasmasse bei der gegebenen Temperatur bezogen auf das jeweilige Scheibenvolumen erfasst wird und von dem aus durch Übergang der Gasmasse der Wärmetransport auf die nächstfolgende Scheibe, symbolisiert durch den Wärmespeicher 17, erfolgt. Soweit sich im Abgas aufgrund exothermer Reaktionen ein Temperaturanstieg ergibt, wird dies im Block 18 berücksichtigt und führt bezüglich des Wärmeinhalts der vom Wärmespeicher Gasmasse 16 auf den Wärmespeicher Gasmasse 17 übertretenden Abgases zu einer gewissen - manchmal im Effekt zum Beispiel im DOC (Diesel Oxidation Catalyst) auch stärkeren - fallweise vernachlässigbaren Temperaturanhebung.

Von der jeweils scheibenbezogen erfassten, strömenden Gasmasse erfolgt der Wärmeübergang auf das Katalysatormaterial unter Berücksichtigung der Wärmeübergangszahl k, wobei das Katalysatormaterial der jeweiligen Scheibe als Wärmespeicher 19 bzw. 20 symbolisiert dargestellt ist. Orientiert am realen Katalysator ergibt sich für den durch die Wärmespeicher 19, 20 symbolisierten Katalysatorkörper zum Umfang des Katalysators hin, entsprechend den gegebenen Temperaturunterschieden, ein Wärmegefälle. Bezogen auf die Darstellung gemäß Fig. 4 und die dort symbolische Andeutung der Ummantelung 5 ist zur Umgebung hin ein Wärmeübergangswiderstand gegeben, in Fig. 4 symbolisiert durch den Wärmeübergangswiderstand R_{C}.

In der durch die Fig. 4 gegebenen Veranschaulichung des thermischen Modells wird davon ausgegangen, dass es, entsprechend den Gegebenheiten im praktischen Betrieb, stationäre und instationäre Betriebsphasen gibt und dass zwischen diesen Betriebsphasen jedenfalls dann unterschieden werden kann und zu unterscheiden ist, wenn die in regelmäßigen Zeitabständen gemessene Abgastemperatur vor Abgasnachbehandlungseinheit, insbesondere also vor Katalysator, sich gegenüber einem zeitlichen Mittelwert über entsprechende Zeitabschnitte ändert. Ist dies nicht der Fall, so wird von einem stationären oder eingeschwungenen Zustand ausgegangen, im anderen Fall von einem instationären Zustand. Entsprechend unterschiedliche Regelkreise kommen zum Einsatz.

Entsprechend dem auf die Temperatur der Abgasnachbehandlungseinheit, die insbesondere als Katalysator ausgebildet ist, im Regelfall kleineren Einfluss des radialen Wärmeübergangs von deren Körper, insbesondere also vom Katalysatorkörper, auf die Umgebung erfolgt im stationären Fall eine Anpassung durch Veränderung des Wärmeübergangswiderstandes R_{C} unter Vermittlung einer Regelanordnung 22. Der stationäre Fall wird entsprechend den vorgeschilderten Gegebenheiten im Modell über eine im Block 21 angedeutete Stationärdetektion ermittelt. Die Regelanordnung 22 berücksichtigt die Differenz der ausgangsseitigen Temperaturen T nach Abgasnachbehandlungseinheit, bezogen auf einen SCR-Katalysator wie im Ausführungsbeispiel also nach Sensor Kat (gemessen), und T nach Modell (gerechnet). Ergeben sich gegenüber über einem zeitlichen Mittelwert der eingangsseitigen, vor der Abgasnachbehandlungseinheit, insbesondere vor dem Katalysator gemessenen Abgastemperatur T relevante Abweichungen, so wird die Wärmeübergangszahl k geändert. Dies, da diese sich in Abhängigkeit von der Strömungsgeschwindigkeit des Abgases, und damit in Abhängigkeit von der Last ändert und zudem der Wärmeinhalt einer jeweiligen Speicherscheibe - wie auch des Körpers der Abgasnachbehandlungseinheit, insbesondere des Katalysators ingesamt - weit mehr von der Temperatur des durchströmenden Abgases als vom Wärmeübergang von der jeweiligen Scheibe auf die Umgebung beeinflusst ist. Die Änderung der Wärmeübergangszahl k folgt ausgehend vom Block 23 in Berücksichtigung der geschilderten dynamischen Faktoren.

Fig. 5 stellt eine Veranschaulichung des vorgeschilderten Ablaufs in einem Blockdiagramm dar, wobei wiederum, als Beispiel auch für andere Abgasnachbehandlungseinheiten, auf einen SCR-Katalysator Bezug genommen ist, so dass T nach KAT oder T vor KAT auch für T nach Abgasreinigungseinheit oder T vor Abgasreinigungseinheit, sei es gemessen oder gerechnet, steht. Gemäß Block 30 erfolgt in der Modellrechnung die dynamische Korrektur der zur Abgasnachbehandlungseinheit austrittsseitigen, gemessenen Abgastemperatur T nach KAT Sensor, die Rechnung der Gastemperaturen und die Rechnung der Temperatur im Katalysatorkörper mit Querverteilung bei Erfassung des Wärmeübergangs zur Umgebung über den Wärmeübergangswiderstand R_{C} und des Wärmeübergangs vom Abgas auf den Katalysatorkörper über die Wärmeübergangszahl k, wobei die Modellrechnung von vorne nach hinten durchgeführt wird.

Die gemessene, eintrittsseitige Abgastemperatur, als T vor KAT bezeichnet, ist im Block 31 erfasst und wird nach Durchlaufen einer Polstellenkompensation gemäß Block 32 in der Modellrechnung gemäß Block 30 verarbeitet. Die gemessene, austrittsseitige Abgastemperatur, als T nach KAT bezeichnet, gemäß Block 33 wird über eine Polstellungskompensation gemäß Block 34 einem Block 35 zugeführt, in dem ein Abgleich der gemessenen Abgastemperatur T nach KAT Sensor gegen die gerechnete Abgastemperatur T nach KAT Modell erfolgt. Bezüglich der gerechneten, austrittsseitigen Abgastemperatur, T nach KAT Modell, erfolgt in Berücksichtigung der Ergebnisse gemäß Block 30 eine gewichtete Mittelwertbildung in Block 36, die als Ergebnis über den Block 37 als gerechnete, austrittsseitige Temperatur, T nach KAT Sensor, dem Block 35 zugeführt wird.

In Berücksichtigung des im Block 35 durchgeführten Abgleichs von gemessener austrittsseitiger Temperatur, T nach KAT Sensor, und gerechneter austrittsseitiger Temperatur, T nach KAT Modell, erfolgt die Unterscheidung zwischen stationärem und instationärem Betriebsverhalten, wobei über die Stationärerkennung gemäß Block 38 und den nachfolgenden Abgleich des Wärmeübergangswiderstandes R_{C} (Block 40) dieser als Parameter in die Modellrechnung gemäß Block 30 eingespeist wird. Bei durch den Abgleich gemäß Block 35 zwischen gemessener und gerechneter, austrittsseitiger Temperatur, T nach KAT, gegebener Instationärerkennung gemäß Block 39 erfolgt der Abgleich der Wärmeübergangszahl k im Block 41 mit nachfolgender Einspeisung in die Modellrechnung gemäß Block 30. In dieser findet auch die gemäß Block 42 zur Verfügung gestellte Umgebungstemperatur T ihre Berücksichtigung.

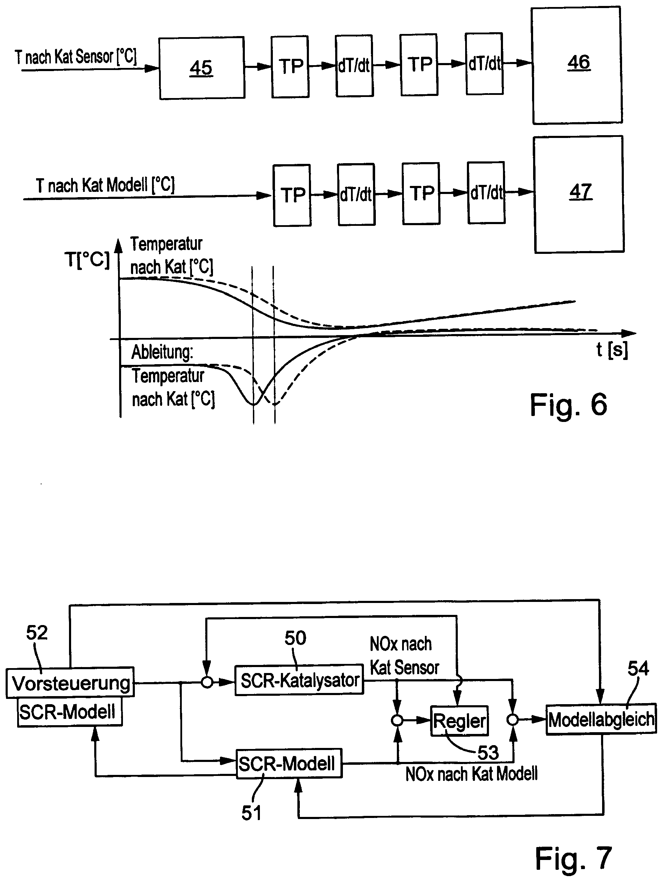

Fig. 6 dient der Veranschaulichtung der im Block 30 berücksichtigten dynamischen Korrektur der zur Abgasnachbehandlungseinheit austrittsseitig gemessenen Temperatur T nach KAT Sensor, also der sensorisch erfassten und damit entsprechend der Sensorträgheit verfälschten Temperaturwerte. Diese werden (strichliert dargestellt)- nach im Block 45 symbolisierter Polstellen-Kompensation, die die Sensorträgheit rechnerisch ausgleicht -, tiefpassgeglättet, in ihrem Verlauf über der Zeit und der dazugehörigen Ableitung, abgetragen. Für den gleichen Zeitabschnitt wird auch die modellbasiert ermittelte Temperatur T nach KAT Modell - also gerechnet - auf entsprechende Weise ermittelt und im Diagramm als durchgehender Linienzug dargestellt. In dem entsprechenden Versatz der zeitlichen Lage der Wendepunkte, entsprechend den Maxima bzw. Minima der Ableitungskurven, stellt sich der Korrekturbedarf dar, wobei im Sinne der Korrektur der gegebene zeitliche Versatz zu minimieren ist. Dies durch entsprechende Änderung des k-Wertes im Sinne einer Verkleinerung oder Vergrößerung in der Modellrechnung. Für die Korrektur des k-Wertes wird dieser zweckmäßigerweise um jeweils die gleiche Größenordnung, zum Beispiel etwa einem Prozentpunkt plus oder minus, korrigiert. Die den Ableitungen entsprechenden Zahlenwerte sind in den Blöcken 46 und 47 erfasst und im Diagramm abgetragen.

Unter Verwendung einer modellbasierten Berechnung der Temperaturverteilung in einem Modell einer Abgasreinigungseinheit, insbesondere unter Verwendung der vorstehend erläuterten, modellbasierten Berechnung wird bei einer Regelstruktur für eine Abgasreinigungseinheit, insbesondere einem SCR-Katalysator gemäß Fig. 7, gearbeitet.

Bezeichnet ist in Fig. 7, wie schon vorstehend auf einen SCR-Katalysator als ein Beispiel auch für anderweitige Abgasreinigungseinheiten abgestellt, mit 50 ein SCR-Katalysator, mit 51 ein SCR-Modell, insbesondere der vorstehend angesprochenen und erläuterten Art, mit 52 eine Vorsteuerung, mit 53 ein Regler und mit 54 ein Modellabgleich.

Im SCR-Modell wird ergänzend, insbesondere parallel zur modellierten Temperaturberechnung, die temperaturabhängige NH₃-Speicherfähigkeit in Abhängigkeit von der NH₃-Konzentration im Abgas berücksichtigt, insbesondere kennfeldbezogen auf Basis von für das jeweilige Katalysatormaterial im Prüfstand ermittelten Daten. In Berücksichtigung dieses Speicherverhaltens und der nachstehend wiedergegebenen Hauptreaktionen wird der Gesamtumsatz an NH₃ ermittelt, der in Korrelation zur Differenz des Anteils an NOₓ vor KAT zum Anteil nach KAT steht und über den sich somit der jeweils als Zielwert angesetzte NOₓ-Wert, zum Beispiel der aufgrund gesetzlicher Vorgaben einzuhaltende Emissionswert bestimmen lässt.

Die angesprochenen Hauptreaktionen sind:
1.) NO + NO₂ + 2 NH₃ → 2 N₂ + 3 H₂O,
   als schnelle Reaktion
2.) 4 NO + 4 NH₃ + O₂ → 4 N₂ + 6 H₂O
   als Standardreaktion, und
3.) 6 NO₂ + 8 NH₃ → 7 N₂ + 12 H₂O,
   als langsame Reaktion.

Für die Modellrechnung kann in Annäherung davon ausgegangen werden, dass die schnellere Reaktion jeweils beendet ist, bevor die langsamere beginnt, so dass rechentechnisch die Reaktionen als nacheinander ablaufend angenommen werden können. Nach der jeweils schnelleren Reaktion werden die umgesetzten Mengen an NO und NO₂ von den Ausgangsmengen abgezogen und nach jeder Reaktion wird die im Volumen einer Scheibe noch verfügbare NH₃-Menge ermittelt.

Bezogen auf die Regelstruktur gemäß Fig. 7 stellt das SCR-Modell 51 somit ein möglichst genaues Abbild des SCR-Katalysators 50 hinsichtlich der verarbeiteten Kenngrößen wie auch der Ausgangswerte, insbesondere des NOₓ-Wertes zur Verfügung.

Dementsprechend sind die Eingangsparameter zum SCR-Modell bezogen auf das über den SCR-Katalysator 50 strömende Abgas: NO, NO₂, Abgasmasse, Temperatur T vor und nach KAT und NH₃. Austrittsseitig werden erfasst: NOₓ, NO/NO₂ umgesetzt, NO/NO₂ maximal umgesetzt, NH₃ gespeichert und NH₃ maximal und gespeichert. In der Vorsteuerung 52 erfolgt die Berechnung des Umsatzbedarfes von NH₃ sowie der ein- und auszuspeichernden NH₃-Menge, aufgeschaltet auf das SCR-Modell 51 und den SCR-Katalysator 50. Dem Regler 53 kommt die Funktion zu, bezogen auf den SCR-Katalysator 50 eine etwaige zusätzliche Dosiermenge zu bestimmen, um den SCR-Katalysator 50 mit dem SCR-Modell 51 fallweise abzugleichen.

Ausgehend davon, dass über die Vorsteuerung auf Basis der Vorgaben des SCR-Modells 51 die jeweils auf den vorgegebenen Emissionswert, also NOₓ nach KAT abgestimmte, als Reduktionsmittel einzudüsende NH₃-Menge berechnet wird und eine entsprechende Eindüsung veranlasst wird, erfolgt über den Modellabgleich 54 eine Auswertung dahingehend, ob zwischen dem SCR-Modell 51 und dem SCR-Katalysator 50 eine größere Abweichung gegeben ist und dies für den Fall, dass in der Vorsteuerung die "Menge Ausspeichern ungleich Null" ist. Ist dies der Fall, so erfolgt im Modell eine Änderung der maximalen Speicherfähigkeit, und damit auch eine Anpassung der Vorsteuerung für den nächsten Lastwechsel, da die Vorsteuerung 52 ihrerseits auf Basis von vom SCR-Modell 51 zur Verfügung gestellten Daten arbeitet. Dies sind: NO nach SCR-Kat ppm, NO₂ nach SCR-Kat ppm, maximal umsetzbares NO mol/s, maximal umsetzbares NO₂ mol/s, NO umgesetzt mol/s, NO₂ umgesetzt mol/s, NH₃ max mol speicherbar, und NH₃ mol gespeichert. Entsprechend der Festlegung, dass der Modell-Abgleich nur dann stattfindet, falls in der Vorsteuerung "Menge Ausspeichern ungleich Null" ist, findet ein Modellabgleich nur in der Phase des Ausspeicherns statt.

Entsprechend den vom SCR-Modell 51 vorgegebenen Daten regelt die Vorsteuerung die eingespritzte NH₃-Menge dahingehend ein, dass der jeweils vorgegebene Emissionswert, also beispielsweise ein gesetzlicher Emissionswert eingehalten wird. In der Vorsteuerung werden hierzu laufend auf Basis der vom SCR-Modell 51 gelieferten Daten der ausgangsseitige NOx-Wert berechnet und mit dem vorgegebenen Emissionswert verglichen. Ergeben sich größere Abweichungen, so wird durch Änderung der durch die Vorsteuerung 52 zugemessenen, einzuspritzenden NH₃-Menge im SCR-Modell 51 die zugeführte NH₃-Menge vergrößert, oder verkleinert.

Zum Beispiel kann ein von der Vorsteuerung 52 errechneter NOₓ-Wert von 0,5 g/kWh zu einem zu großen Umsatz an NOₓ bezogen auf einen Emissionswert, so beispielsweise den gesetzlichen Emissionswert von 0,67 g/kWh, führen. Dementsprechend wird von der Vorsteuerung 52 die Einspritzung von NH₃ zurückgenommen. Ist der Umsatz an NOₓ kleiner als der vorgegebene Emissionswert, so veranlasst die Vorsteuerung 52 die Vergrößerung der NH₃-Einspritzmenge. Die Einspeicherung von NH₃ wird so vorgenommen, dass eine Schlupfgefahr ausgeschlossen ist.

Über den Modellabgleich 54 wird zu definierten Zeitpunkten der zeitliche Verlauf zwischen einem sich real einstellenden NOₓ nach KAT und dem NOₓ gerechnet nach Modell erfasst. Ergibt sich zwischen diesen Werten eine Differenz und werden diese Werte in einem Diagramm als NOₓ-Werte über der Zeit in Kurven aufgetragen, so ist die Fläche zwischen den Kurven ein Maß für den Modellfehler. Wenn dieser Modellfehler einen Schwellwert übersteigt, wird die Speicherfähigkeit im Modell geändert. Solche Änderungen werden erfindungsgemäß bevorzugt nur während des Ausspeicherns vorgenommen, da die Speicherfähigkeit sich durch Kat-Alterung nur langsam ändert und Modellfehler zudem im Vergleich zum Einspeichern deutlicher sichtbar werden. In Verbindung mit einer solchen, über den Modellabgleich 54 erfolgenden Korrektur wird auch die Alterung des Katalysators bevorzugt automatisch berücksichtigt.

Ob unter den jeweiligen Gegebenheiten das SCR-Modell 51 auch hinsichtlich seiner maximalen Speicherfähigkeit korrigiert werden muss, also eine Erhöhung oder Verkleinerung der Speicherfähigkeit erforderlich ist, hängt von den sensorisch ermittelten, also gemessenen NOₓ-Werten ab. Es gilt: NOₓ-Modell - NOₓ real > 0:Speicherfähigkeit des Modells erhöhen, < 0:Speicherfähigkeit des Modells verkleinern.

Dem Regler 53 kommt die Funktion zu, im stationären Betrieb bei kleineren Abweichungen der im SCR-Modell 51 errechneten NOₓ-Werte von den gemessenen NOₓ-Werten nach SCR-Katalysator 50 eine Anpassung der eingedüsten NH₃-Masse überlagert zu und unabhängig von den Festlegungen der Vorsteuerung 52 gegebenenfalls vorzunehmen, um eine schnelle Anpassung im Hinblick auf die Einhaltung der NOₓ-Emissionswerte zu gewährleisten.

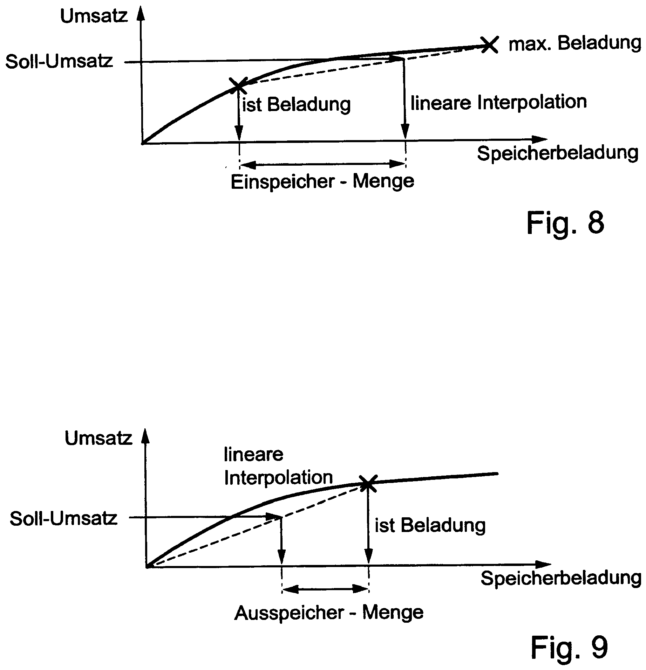

Diese Anpassung erfolgt bevorzugt dadurch, dass der NOₓ- oder NH₃-Umsatz über der Speicherbeladung erfasst wird und Abweichungen der Ist-Beladung von der dem Sollumsatz entsprechenden Beladung als Maßstab dafür erfasst werden, in welcher Menge einzuspeichern oder auszuspeichern ist. Im Falle der Einspeicherung entspricht die einzuspeichernde Menge der Differenz zwischen Ist-Beladung und dem Sollumsatz entsprechender Beladung bezogen auf eine lineare Interpolation der Beladungskurve zwischen Ist-Beladungswert und maximaler Beladung. Im Falle des Ausspeicherns entspricht die auszuspeichernde Menge der Differenz der Ist-Beladung und einer linearen Interpolation der Beladungskurve zwischen Ist-Beladung und deren Durchgang durch den Kreuzungspunkt der Achsen, wie in den Fig. 8 und 9 veranschaulicht.

**Bezugszeichenliste**

| | | |
|---|---|---|
| 1 | Scheibe | |
| 2 | Scheibe | |
| 3 | Scheibe | |
| 4 | Scheibe | |
| 5 | Ummantelung | |
| 6 | Segment | |
| 7 | Quader | |
| 8 | Sensor | |
| 9 | Sensor | |
| 10 | Sensor | |
| | | |
| 15 | Polstellenkompensation | |
| 16 | Wärmespeicher-Gasmasse | |
| 17 | Wärmespeicher-Gasmasse | |
| 18 | Block | |
| 19 | Wärmespeicher | |
| 20 | Wärmespeicher | |
| 21 | Block Stationärdetektion | |
| 22 | Regelanordnung | |
| 23 | Block - Dynamik gemäß Fig. 6 | |
| | | |
| Blöcke | | |
| 31 | T vor KAT (gemessene Temp) | |
| 32 | Polstellenkompensation | |
| 33 | T nach KAT (gemessene Temp) | |
| 34 | Polstellenkompensation | |
| 35 | Temperaturabgleich T nach KAT gemessen/T nach KAT gerechnet) | |
| 36 | Mittelwertbildung | |
| 37 | Block | |
| 38 | Stationärerkennung | |
| 39 | Instationärerkennung | |
| 40 | Abgleich R_{C} | |
| 41 | Abgleich k | |
| 42 | Umgebungstemperatur | |
| 45 | Polstellenkompensation | |
| 46 | Zahlenwerte | |
| 47 | Zahlenwerte | |
| | | |
| 50 | SCR-Katalysator | ) |
| 51 | SCR-Modell | ) |
| 52 | Vorsteuerung | ) Regelstruktur |
| 53 | Regler | ) |
| 54 | Modellabgleich | ) |

m Massenanteile
n Stoffmengenanteile
***ṁ*** Gas
Tgas
***ṁ*** NO [kg/h]
***ṁ*** NO₂ ,,
***ṁ*** NH₃
***n*** NO [g/mol]
***n*** NO₂ [g/mol]
***n*** NH₃ [g/mol]
***n*** NH₃Scheibe [g/mol]
R_{C} Wärmeübergangswiderstand
T nach KAT Sensor (gemessen)
T nach KAT Modell (gerechnet)
T vor SCR (gemessen)
k Wärmeübergangszahl

## Patentansprüche

1. Verfahren zur modellbasierten Bestimmung der Temperaturverteilung einer Nachbehandlungseinheit für Abgase, insbesondere eines Katalysators, auch als SCR-Katalysator, oder eines Partikelfilters, mit axial vom Abgas durchströmten und im Modell der Nachbehandlungseinheit zumindest axial segmentierter Ausbildung derselben, axialem Wärmeübergang zwischen den Segmenten zumindest vorwiegend über das Abgas sowie radialem Wärmeübergang vom Umfang der Nachbehandlungseinheit auf die Umgebung,
**dadurch gekennzeichnet,**
**dass**, im Modell, in Abhängigkeit davon, ob sich die vor der Nachbehandlungseinheit in vorgegebenen, insbesondere gleichen Zeitabständen gemessenen Abgastemperaturen gegenüber einem zeitlichen Mittelwert derselben ändern, zwischen einem stationären und einem instationären Arbeitsbetrieb unterschieden wird, dass ferner die Berechnung des radialen Wärmeübergangs auf den Umfang der Nachbehandlungseinheit unter Berücksichtigung eines Wärmeübergangswiderstandswertes (R_{C}) und die Berechnung des Wärmeübergangs vom Abgas auf die Segmente der Nachbehandlungseinheit unter Berücksichtigung einer Wärmeübergangszahl (k) erfolgt und dass Abweichungen der im Modell errechneten Temperatur nach Nachbehandlungseinheit von der nach der Nachbehandlungseinheit gemessenen, gemittelten Temperatur im stationären Arbeitsbetrieb durch Anpassung des Wärmeübergangsbeiwertes (R_{C}) und im instationären Arbeitsbetrieb durch Anpassung der Wärmeübergangszahl (k) berücksichtigt werden.

2. Verfahren nach Anspruch 1,
**dadurch gekennzeichnet,**
**dass** für die Modellierung bezogen auf eine axiale Segmentierung der Nachbehandlungseinheit in Scheiben und eine radiale Segmentierung der Scheiben, insbesondere in Ringe, die mittlere gerechnete Temperatur nach Nachbehandlungseinheit auf Basis der für die Flächenanteile der axial letzten Scheibe gerechneten Temperaturen gemittelt bestimmt wird.

3. Verfahren nach Anspruch 1 oder 2,
**dadurch gekennzeichnet,**
**dass** für einen von einem gegebenen Betriebszustand ausgehenden stationären Arbeitsbetrieb in der Modellrechnung die für diesen Betriebszustand gegebene Wärmeübergangszahl (k) beibehalten wird.

4. Verfahren nach einem der vorhergehenden Ansprüche,
**dadurch gekennzeichnet,**
**dass** für einen von einem gegebenen Betriebszustand ausgehenden instationären Arbeitsbetrieb in der Modellierung für diesen Betriebszustand mit einem gleichbleibenden Wärmeübergangsbeiwert (R_{C}) gerechnet wird.

5. Verfahren nach einem der vorhergehenden Ansprüche,
**dadurch gekennzeichnet,**
**dass** der Wärmeübergangsbeiwert (R_{C}) von den Umgebungsbedingungen zur Nachbehandlungseinheit abhängig ist.

6. Verfahren nach einem der vorhergehenden Ansprüche,
**dadurch gekennzeichnet,**
**dass** die Änderungsrichtung der für den instationären Arbeitsbetrieb veränderlichen Wärmeübergangszahl (k) dadurch bestimmt wird, dass die gemessenen und errechneten mittleren Temperaturverläufe nach Nachbehandlungseinheit über der Zeit erfasst und die Ableitungen hierzu gebildet werden, wobei in Abhängigkeit von der relativen zeitlichen Lage der der Maxima und Minima der Ableitungen entsprechenden Wendepunkte die Wärmeübergangszahl (k) vergrößert und verkleinert wird.

7. Verfahren zur modellbasierten Bestimmung der Temperaturverteilung einer Nachbehandlungseinheit für Abgase unter Verwendung eines SCR-Katalysators nach einem oder mehreren der vorhergehenden Ansprüche, bei dem der SCR-Katalysator (50) in eine Regelstruktur mit zumindest einem SCR-Modell (51), einer Vorsteuerung (52) und einem Regler (53) eingebunden ist, wobei über die Vorsteuerung (52) die dem SCR-Katalysator (50) zuzuführende NH₃-Menge auf die Einhaltung eines Emissionswertes, insbesondere eines Emissionsgrenzwertes, ausgerichtet, berechnet und/oder eingestellt wird, wobei das SCR-Modell (51) die Eingangsgrößen für die Vorsteuerung (52) liefert, wobei in der Vorsteuerung (52) laufend der den gelieferten Daten entsprechende NOₓ-Wert berechnet und mit dem vorgegebenen Emissionswert verglichen wird und wobei der Regler (53) durch Anpassung der jeweiligen, dem SCR-Katalysator (50) zugeführten NH₃-Menge den nach SCR-Katalysator (50) gemessenen NOₓ-Istwert an den errechneten NOₓ-Sollwert nach SCR-Katalysator (50) angleicht.

8. Verfahren nach Anspruch 7,
**dadurch gekennzeichnet,**
**dass** die Angleichung im stationären Betrieb schrittweise zum Abgleich kleinerer Abweichungen des nach SCR-Katalysator (50) gemessenen NOₓ-Istwertes vom errechneten NOₓ-Sollwert nach SCR-Katalysator (50) vorgenommen wird.

9. Verfahren nach Anspruch 7 oder 8,
**dadurch gekennzeichnet,**
**dass** als Eingangsgrößen für die Vorsteuerung (52) insbesondere vorgesehen sind: NO und NO₂ nach SCR ppm, bei der gegebenen Last maximal umsetzbares NO und NO₂ mol/s, umgesetztes NO und NO₂ mol/s, maximales NH₃-Speichervermögen NH₃ max. mol speicherbar und die NH₃-Speicherbeladung NH₃ mol gespeichert.

10. Verfahren nach einem der Ansprüche 7 bis 9,
**dadurch gekennzeichnet,**
**dass** größenabhängig zwischen dem in der Vorsteuerung (52) auf Basis der Eingangsgrößen ermittelten NOₓ-Wert und dem vorgegebenen Emissionswert auftretende Abweichungen durch Änderung in der Zuführung von NH₃ berücksichtigt werden, derart, dass sich für das im SCR-Modell (51) eingespeicherte NH₃ eine Vergrößerung oder Verringerung ergibt.

11. Verfahren nach einem der Ansprüche 7 bis 10,
**dadurch gekennzeichnet,**
**dass** in die Regelstruktur ein Modellabgleich (54) integriert ist, in dem zu definierten Zeitpunkten der zeitliche Ablauf der Differenz zwischen gemessenen und im Modell erfassten NOₓ-Werten als Maß für Modellfehler erfasst wird.

12. Verfahren nach einem der Ansprüche 7 bis 11,
**dadurch gekennzeichnet,**
**dass** die NOₓ-Reaktionen im SCR-Modell (53) über nachstehende Hauptreaktionen
1.) NO + NO₂ + 2 NH₃ → 2 N₂ + 3 H₂O,
als schnelle Reaktion
2.) 4 NO + 4 NH₃ + O₂ → 4 N₂ + 6 H₂O
als Standardreaktion, und
3.) 6 NO₂ + 8 NH₃ → 7 N₂ + 12 H₂O,
als langsame Reaktion.
erfasst werden.

## Claims

1. Method for the model-based determination of the temperature distribution of an exhaust gas post-treatment unit, in particular of a catalytic converter, also referred to as an SCR catalytic converter, or of a particle filter, with a design of said post-treatment unit through which exhaust gas flows axially and which is segmented at least axially in the model of the post-treatment unit, an axial heat transfer between the segments, at least predominantly by means of the exhaust gas, and a radial heat transfer from the circumference of the post-treatment unit to the surroundings, **characterized**
**in that**, depending on whether the exhaust gas temperatures which are measured upstream of the post-treatment unit at predetermined, in particular equal time intervals change with respect to a chronological mean value thereof, a differentiation is made in the model between a steady-state and a non-steady-state operating mode, in that, in addition, the calculation of the radial heat transfer to the circumference of the post-treatment unit is carried out taking into account a heat transfer resistance value (R_{C}) and the calculation of the heat transfer from the exhaust gas to the segments of the post-treatment unit is carried out taking into account a heat transfer coefficient (k), and in that deviations of the temperature downstream of the post-treatment unit, calculated in the model, from the averaged temperature, measured downstream of the post-treatment unit, are taken into account in the steady-state operating mode by adapting the heat transfer coefficient (R_{C}), and in the non-steady-state operating mode by adapting the heat transfer coefficient (k).

2. Method according to Claim 1,
**characterized**
**in that**, for the modeling with respect to axial segmentation of the post-treatment unit into disks and radial segmentation of the disks, in particular into rings, the average calculated temperature downstream of the post-treatment unit is determined by averaging on the basis of the temperatures calculated for the area portions of the last disk in the axial direction.

3. Method according to Claim 1 or 2,
**characterized**
**in that**, for a steady-state operating mode which is based on a given operating state, the given heat transfer coefficient (k) for this operating state is retained in the model calculation.

4. Method according to one of the preceding claims,
**characterized**
**in that**, for a non-steady-state operating mode which is based on a given operating state, a constant heat transfer coefficient (R_{C}) is expected in the modeling for this operating state.

5. Method according to one of the preceding claims,
**characterized**
**in that** the heat transfer coefficient (R_{C}) is dependent on the ambient conditions relating to the post-treatment unit.

6. Method according to one of the preceding claims, **characterized**
**in that** the direction of the change in the heat transfer coefficient (k), which is variable for the non-steady-state operating mode, is determined by virtue of the fact that the measured and calculated average temperature profiles downstream of the post-treatment unit plotted against the time are obtained and the derivatives thereof are formed, wherein the heat transfer coefficient (k) is increased and decreased as a function of the relative timing of the inflection points which correspond to the maximum and minimum values of the derivatives.

7. Method for the model-based determination of the temperature distribution of an exhaust gas post-treatment unit using an SCR catalytic converter, according to one or more of the preceding claims, in which the SCR catalytic converter is integrated into a control structure with at least one SCR model (51), a pilot controller (52) and a regulator (53), wherein the quantity of NH₃ which is to be fed to the SCR catalytic converter (50) is tailored, calculated and/or set so as to comply with an emission value, in particular an emission limiting value, by means of the pilot controller (52), wherein the SCR model (51) supplies the input variables for the pilot controller (52), wherein in the pilot controller (52) the NOₓ value corresponding to the supplied data is continuously calculated and compared with the predefined emission value and wherein, by adapting the respective quantity of NH₃ fed into the SCR catalytic converter (50), the regulator (53) approximates the NOx actual value measured downstream of the SCR catalytic converter (50) to the calculated NOx setpoint value downstream of the SCR catalytic converter (50).

8. Method according to Claim 7,
**characterized**
**in that** the approximation is performed incrementally in the steady-state operating mode in order to compensate relatively small deviations of the NOx actual value, measured downstream of the SCR catalytic converter (50), from the calculated NOx setpoint value downstream of the SCR catalytic converter (50).

9. Method according to Claim 7 or 8,
**characterized**
**in that** input variables which are provided for the pilot controller (52) are, in particular: NO and NO₂ downstream of the SCR ppm, the maximum amount of NO and NO₂ mol/s which can be converted at the given load, the converted NO and NO₂ mol/s, the maximum NH₃ storage capacity NH₃ max. mol which can be stored, and the NH₃ storage load NH₃ mol stored.

10. Method according to one of Claims 7 to 9,
**characterized**
**in that** deviations which occur in a variable-dependent fashion between the NOₓ value determined on the basis of the input variables in the pilot controller (52) and the predefined emission value are taken into account by changing the supply of NH₃ in such a way that an increase or decrease occurs for the NH₃ stored in the SCR model (51).

11. Method according to one of Claims 7 to 10,
**characterized**
**in that** a model adjustment means (54), in which the time sequence of the difference between measured NOₓ values and those determined in the model is determined as a measure of model errors, is integrated into the control structure.

12. Method according to one of Claims 7 to 11,
**characterized**
**in that** the NOₓ reactions in the SCR model (53) are determined by means of subsequent main reactions
1.) NO + NO₂ + 2 NH₃ → 2 N₂ + 3 H₂O,
as a fast reaction
2.) 4 NO + 4 NH₃ + O₂ → 4 N₂ + 6 H₂O
as a standard reaction, and
3.) 6 NO₂ + 8 NH₃ → 7 N₂ + 12 H₂O,
as a slow reaction.

## Revendications

1. Procédé pour déterminer sur la base d'un modèle la distribution de température dans une unité de traitement de gaz d'échappement, notamment dans un catalyseur, par exemple un catalyseur appelé SCR, ou un filtre à particules, traversés axialement par les gaz d'échappement et segmentés au moins axialement dans le modèle de l'unité de traitement, avec un transfert de chaleur axial entre les segments de manière prédominante par l'intermédiaire des gaz d'échappement et un transfert de chaleur radial entre la périphérie de l'unité de traitement et l'environnement, **caractérisé en ce que**
selon que la température des gaz d'échappement mesurées à des intervalles de temps prédéterminés et en particulier identiques varient en amont de l'unité de traitement par rapport à une valeur moyenne temporelle, le modèle fait la distinction entre un régime de travail stationnaire et un régime de travail non stationnaire,
**en ce qu'**en outre, le calcul du transfert de chaleur radial à la périphérie de l'unité de traitement s'effectue en tenant compte d'un coefficient (R_{C}) de résistance au transfert de chaleur et le calcul du transfert de chaleur entre les gaz d'échappement et les segments de l'unité de traitement s'effectue en tenant compte d'un coefficient (k) de transfert de chaleur et
**en ce que** les écarts entre la température en aval de l'unité de traitement calculée dans le modèle et la température moyenne mesurée en aval de l'unité de traitement sont pris en compte en régime de travail stationnaire par adaptation du facteur de transfert de chaleur (R_{C}) et en régime de travail non stationnaire par adaptation du coefficient (k) de transfert de chaleur.

2. Procédé selon la revendication 1, **caractérisé en ce que** dans la modélisation, la température moyenne calculée en aval de l'unité de traitement sur la base d'une segmentation axiale de l'unité de traitement en disques et d'une segmentation radiale des disques en particulier en anneaux est déterminée sur la base des températures calculées sur les fractions de surface du disque situé en dernier dans la direction axiale.

3. Procédé selon les revendications 1 ou 2, **caractérisé en ce que** dans la modélisation, pour un régime de travail stationnaire à partir d'un état de fonctionnement donné, le coefficient (k) de transfert de chaleur donné pour cet état de fonctionnement est conservé.

4. Procédé selon l'une des revendications précédentes, **caractérisé en ce que** dans la modélisation, pour un régime de travail non stationnaire à partir d'un état de fonctionnement donné, le calcul s'effectue pour cet état de fonctionnement avec un facteur de transfert de chaleur constant.

5. Procédé selon l'une des revendications précédentes, **caractérisé en ce que** le facteur de transfert de chaleur dépend des conditions de l'environnement de l'unité de traitement.

6. Procédé selon l'une des revendications précédentes, **caractérisé en ce que** la direction de la modification du coefficient (k) de transfert de chaleur variable en régime de travail non stationnaire est déterminée en saisissant l'évolution au cours du temps de la température moyenne mesurée et de la température moyenne calculée en aval de l'unité de traitement, en en formant la dérivée et en augmentant ou en diminuant le coefficient (k) de transfert de chaleur en fonction de la position dans le temps des points d'inversion qui correspondent aux maxima et aux minima des dérivées.

7. Procédé de détermination sur la base d'un modèle de la répartition des températures d'une unité de traitement de gaz d'échappement qui recourt à un catalyseur SCR selon l'une ou plusieurs des revendications précédentes, dans lequel le catalyseur SCR (50) intervient dans une structure de régulation présentant au moins un modèle de SCR (51), une précommande (52) et un régulateur (53), la quantité de NH₃ à apporter au catalyseur SCR (50) par l'intermédiaire de la précommande (52) étant alignée, calculée et/ou ajustée de manière à respecter un indice d'émission et en particulier une valeur limite d'émission, le modèle SCR (51) fournissant les grandeurs d'entrée à la précommande (52), une valeur de NOₓ correspondant aux données fournies étant calculée en permanence dans la précommande (52) et étant comparée à l'indice d'émission prédéterminée, le régulateur (53) amenant la valeur effective des NOₓ mesurée en aval du catalyseur SCR (50) à la valeur de consigne calculée des NOₓ en aval du catalyseur SCR (50) en adaptant la quantité de NH₃ apportée au catalyseur SCR (50).

8. Procédé selon la revendication 7, **caractérisé en ce que** en fonctionnement stationnaire, l'égalisation visant à amener la valeur effective des NOₓ mesurée en aval du catalyseur SCR (50) à la valeur de consigne calculée des NOₓ en aval du catalyseur SCR (50) s'effectue pas-à-pas.

9. Procédé selon les revendications 7 ou 8, **caractérisé en ce que** les grandeurs d'entrée prévues pour la précommande (52) sont en particulier : le NO et le NO₂ en aval du SCR en ppm, le NO et le NO₂ maximum réagis pour une charge donnée en moles/s, le NO et le NO₂ non réagis en moles/s, la capacité maximale d'accumulation de NH₃ en moles et la charge de la réserve de NH₃ en moles de NH₃ conservées.

10. Procédé selon l'une des revendications 7 à 10, **caractérisé en ce que** les écarts survenant en fonction de la taille entre la valeur de NOₓ déterminée dans la précommande (52) sur la base des grandeurs d'entrée et la valeur d'émission prédéterminée sont pris en compte en modifiant l'apport de NH₃ de telle sorte que l'on obtienne une augmentation ou une diminution du NH₃ conservé dans le modèle SCR (51).

11. Procédé selon l'une des revendications 7 à 10, **caractérisé en ce que** dans la structure de régulation est intégrée une équation de modélisation (54) dans laquelle à des instants définis, l'évolution dans le temps de la différence entre les valeurs de NOₓ mesurées et saisies dans le modèle est saisie en tant de mesure de l'erreur de modélisation.

12. Procédé selon l'une des revendications 7 à 10, **caractérisé en ce que** les réactions de production de NOₓ utilisées dans le régulateur (53) sont les réactions principales suivantes :
1.) NO + NO₂ + 2 NH₃ -> 2 N₂ + 3 H₂O, réaction rapide,
2.) 4 NO + 4 NH₃ + O₂-> 2 N₂ + 6 H₂O, réaction standard, et
3.) 6 NO₂ + 8 NH₃ -> 7 N₂ + 12 H₂O, réaction lente.
